Europäisches Patentamt

European Patent Office

Office européen des brevets

(19)

(11) Publication number: **0 154 687 B1**

(12) **EUROPEAN PATENT SPECIFICATION**

(45) Date of publication of patent specification: **22.04.92**   (51) Int. Cl.⁵: **B01L  3/00**, G01N 33/53, G01N 33/545

(21) Application number: **84114261.5**

(22) Date of filing: **18.02.83**

(60) Publication number of the earlier application in accordance with Art.76 EPC: **0 087 899**

(54) **Carrier plate and screening assembly for immunoassay procedures.**

(30) Priority: **03.03.82 US 354554**

(43) Date of publication of application:
**18.09.85 Bulletin  85/38**

(45) Publication of the grant of the patent:
**22.04.92 Bulletin  92/17**

(84) Designated Contracting States:
**BE DE FR GB IT NL SE**

(56) References cited:
**EP-A- 0 040 943        EP-A- 0 042 755**
**FR-A- 2 277 563        FR-A- 2 362 397**
**FR-A- 2 369 557        US-A- 4 111 754**
**US-A- 4 200 613**

(73) Proprietor: **Becton, Dickinson and Company**
**Mack Centre Drive P.O. Box 2224**
**Paramus New Jersey 07652-1149(US)**

(72) Inventor: **Sapatino, Bruno V.**
**1401 S. Port Dr.**
**Oxnard, CA 93030(US)**
Inventor: **Johnson, Luther R.**
**878 Capitan Street**
**Thousand Oaks, CA 91320(US)**

(74) Representative: **Ruffles, Graham Keith et al**
**MARKS & CLERK 57-60 Lincoln's Inn Fields**
**London WC2A 3LS(GB)**

## Description

The present invention relates to a multi-well screening system useful in immunoassay procedures, and more particularly, concerns such a screening assembly suitable for the screening of hydbrid cell cultures such as hybridomas. The present invention also relates to a carrier plate useful in immunoassay procedures and having elements thereof adapted to absorb immunogenic substances thereto.

Monoclonal antibodies are being investigated by researchers for a variety of uses, including the treatment of cancer. It is now well known that monoclonal antibodies can be produced in large quantities from hybridomas. A hybridoma is a hybrid cell which has been produced by joining together a cancer cell with an antibody-making cell. Inasmuch as these hybridomas can produce specific antibodies in large quantities, it is desirable to be able to detect and quantify the presence of the specific hybridoma under investigation.

The search for a certain hybridoma can be very tedious and time-consuming. In the research laboratory, the quest for a certain antibody may start with the injection of an antigen, against which the certain antibody will form, into a mouse. Following removal of the mouse's spleen, the spleen cells are fused with cancer cells. It is to be appreciated that after this process of fusing cancer cells and spleen cells, the researcher must be able to detect which of these fused cells are actually producing the desired antibodies. Only a very small percentage of the cells which the researcher started with will actually fuse together to form a hybrid. This search for fused cells usually includes the distribution of the combined spleen cells and cancer cells into various compartments in order to make the proper identification. Eventually, the researcher attempts to isolate not only the hybridomas, but also to isolate those hybridomas which are producing the sought for antibody. Once again, the researcher may rely upon various compartmentalized trays or tubes in order to complete this isolation.

In the detection and quantification of the various proteins, such as monoclonal antibodies produced by hybridomas, the researcher may rely upon one or more assays. For example, he may use the enzyme-linked immunosorbent assay (ELISA) or the radioimmunoassay (RIA). These immunoassays are very well known to researchers in this field. Both of these assays may utilize test equipment including multiple compartments or wells so that processing of each may be performed simultaneously on a microtiter basis. For example, Schlieicher and Schuell, Inc, Keene, New Hampshire 03431, USA, utilize a 96 place microsample filtration manifold which has direct application in the DNA/RNA hybridization research field. Also, Klebe, Robert J, et al, in "Replica Plating of Cultured Mammalian Cells," Somatic Cell Genetics, Vol 7, No 3, 1981, pp 271-180, describe a device which permits the replica plating of lymphocytes as well as substrate adherent fibroblasts. In this study, the authors relied on ELISA assays with 96 well microtiter plates.

Beads have been utilized in these assay procedures in order to link antigen or antibody thereto in the detection of the sought for biological substance. For example, Litton Bionetics Laboratory Products, Kensington, Maryland, USA, have described the use of a "BIO-BEAD" which is used for the rapid screening of serum specimens for the presence of detectable antibodies to the TORCH group of antigens. These beads include a magnetic component for use in the Litton Bionetics' system. Micro-Media Systems, Inc, Potomac, Maryland, USA, have disclosed an MIC and bacterial identification device which includes a tray with a multiplicity of wells and a cover with downwardly depending pins which fit in alignment in the wells. These pins are adapted to permit protein (antigen or antibody) to become adsorbed to the surface thereof. It has been found, however, that elongate pins may cause a capilliary action when dipped into fluid in the wells. As a result, accuracy of fluid transferred to the pin may be compromised. Furthermore, fluid sometimes drips from these pins after the cover is removed from the tray with the wells. This may result in cross-contamination of the fluid into adjacent wells.

Thus, while the above-mentioned devices may be known and used, improvements are being sought in the multiple-well approach to screening of biological substances, such as hybridomas. It is to such improvements that the present invention is directed.

The multi-well screening assembly of the present invention is useful in immunoassay procedures and comprising a tray adapted to hold fluid therein. A carrier plate is positioned on the tray which includes a plurality of separated, individualized pockets depending into the tray. Each pocket contains a removable body therein each removable body being substantially spherical or disc shaped and having a surface adapted to adsorb an immunogenic substance thereto. Each pocket also includes means to allow fluid to enter into the pocket while the body remains therein and to allow the removal of the body therefrom. Each pocket further includes a lower opening which is smaller than the removable body deposited in the pocket and which allows fluid to enter in to the pocket while the removeable body is in the pocket.

In still another aspect of the present invention, a carrier plate, useful in immunoassay procedures, comprises the carrier plate with pockets therein substantially as defined above.

In accordance with the present invention, simultaneous processing of all wells of a microtiter for biological substance screening purposes can be achieved. By employing an immunoassay, such as ELISA or RIA or the like, the present invention allows the determination of those wells which contain hybridoma cells which produce antibodies. It is also desirable to utilize the present invention in determining which of the wells of the screening assembly contain hybridoma cells and also contain specific monoclonal antibodies. The present invention allows the handling of all wells, preferably 96, in one step instead of much smaller numbers of wells which are presently being handled by researchers. In addition, in utilizing the beads or disks of the present invention, only an extremely small amount of fluid is withdrawn from the wells upon completion of the individual tests, that is, only the fluid that is retained on the beads or disks is removed from the wells. It is appreciated that the simultaneous transfer of up to 96 protein adsorbing substrate units to various other multi-well or multi-tube devices contributes to saving material and labour in the performance of immunoassay tests. The structure of the present invention desirably eliminates or minimizes cross-contamination among the plurality of wells during the simultaneous transfer of the aseptic fluids. The components of the present invention may also be made inexpensively so as to be disposable after use.

The present invention will now be illustrated by way of example with reference to the accompanying drawings, in which:

Fig 1 is a perspective view illustrating a multi-well screening assembly with the preferred tray suitable for use in the present invention and carrier plate separated from each other as they may appear before use;

Fig 2 is a side elevation, partly in section, illustrating the assmebled components of the arrangement of Fig 1;

Fig 2a is a view similar to Fig 2 illustrating an alternative tray element;

Fig 13 is a perspective view of carrier plate embodiment of the present invention;

Fig 14 is a side elevation, partly in section, of the carrier plate of Fig 13;

Fig 15 is a side elevation, partly in section, of the carrier plate of Fig 13 assembled onto a tray similar to the tray shown in Fig 1;

Fig 16 is a side elevation, partly in section, of the carrier plate of Fig 13 assembled onto a tray for washing or the like without having individualized wells therein;

Fig 17 is a side elevation, partly in section, illustrating a rack of tubes corresponding in geometry to the carrier plate of Fig 13 shown placed on top of that carrier plate;

Fig 18 is a side elevation, partly in section, illustrating the assembly of Fig 17 inverted to allow the beads to fall into the tubes.

There are shown in the drawings and will herein be described in detail some preferred, non-limiting embodiments of the invention.

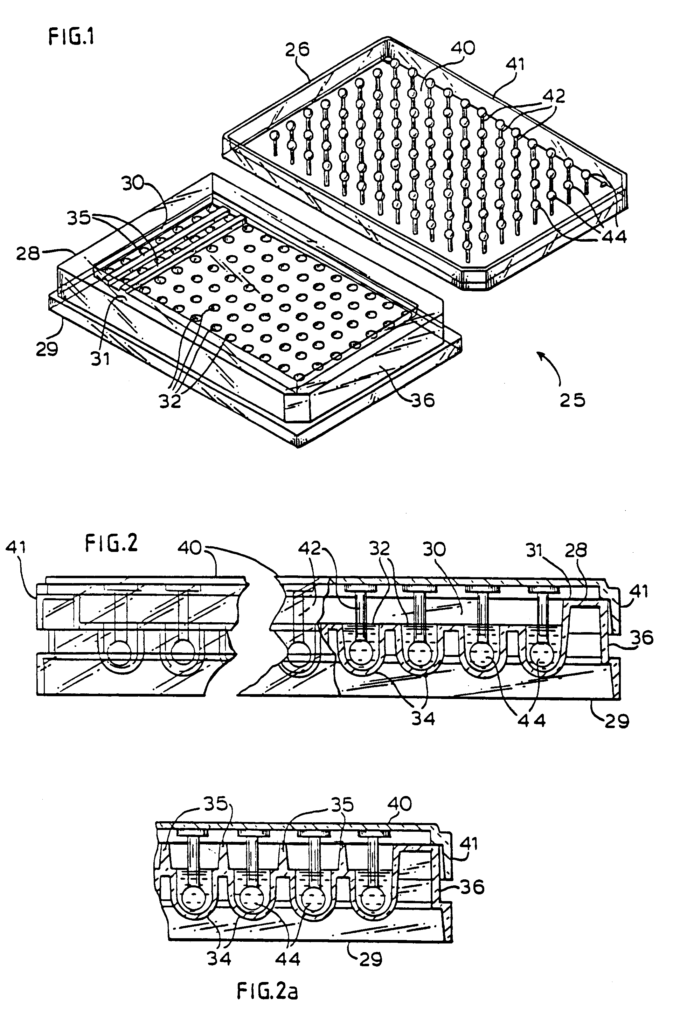

Adverting now to the drawings, and Fig 1 and 2 in particular, there is illustrated a multi-well screening assembly 25 which is useful in immunoassay procedures. This assembly is comprised of two general components: a carrier plate 26 and a tray 28 onto which the carrier plate is positined during use, as more clearly illustrated in Fig 2.

Tray 28 generally has a substantially planar body 29 which may be supported on a flat surface. Formed within body 29 is a deep recess 30 so that a web 31 surrounds the recess. Formed within recess 30 is a plurality of separated, individualized wells 32, which preferably have cylindrically shaped sidewalls and include a spherically shaped bottom 34 inside the tray body so that fluid may be deposited therein. Wells 32 are preferably formed in the tray in an orthogonal array of rows and columns. In the arrangements being described, there are 96 wells in the tray, with 12 rows each with 8 wells. The individualized wells are provided in the tray to minimize the volume of solution required to coat beads 44. The wells may all be placed in the bottom of one large recess 30 as shown in Fig 2, or each row may be separated with barriers 35 as shown in Fig 2a, or one or two rows only may be separated as seen in Fig 1. The advantage of using a single recess is that it requires only the one-time addition of a fixed volume of solution to submerge all wells. The advantage of separated rows is that the assembly allows for use of individual rows for controls; however, it requires the individual addition of solution to each row. The separation of one or two rows only is a compromise which provides both control provisions and reduced filling operations.

While the volume of each well may vary according to many factors, it is preferred, in the arrangement being described, that each well have the capacity to hold approximately one hundered microlitres of fluid.

In order to minimize expense and render the tray disposable, it is preferred that it be made of plastic material. To minimize the amount of solution used, it is also preferred that the material selected for the tray be made of a non-protein adsorbent material such as polypropylene, polyethylene or the like.

Turning now to carrier plate 26, it is comprised of generally planar support body 40 surrounded by a raised wall 41. The dimensions of wall 41 allow the carrier plate to be positioned on tray 28 so that wall 41 surrounds wall 36 on the tray, as more clearly seen in Fig 2. Protruding from the planar surface of body 40 is a plurality of posts 42. These posts are arranged in the same pattern as wells 32 in the tray and generally correspond to the same number of wells. In the arrangement being described there are thus 96 posts orthogonally arranged in 12 rows of 8 posts each.

When the carrier plate is positioned onto the tray as illustrated in Fig 2, each post depends into one of the wells in the tray. It is preferred that the posts all have the same diameter and length so that consistency and uniformity can be maintained. In this arrangement a substantially spherical bead 44 is connected to the distal end of each post so that the bead depends deepest into the well when the carrier plate is positioned on the tray. In order to allow the beads to enter the wells, it is preferred that they have a diameter of about 3.2 millimeters, although this size may vary according to circumstances.

Carrier plate 26 is preferably an integrally formed or molded structure. Plastics are the materials of choice, such as styrene acrylonitrile, polypropylene, polyethylene, polystyrene and the like. However, irrespective of the material of carrier plate 26 in general, beads 44 must be made from materials which will allow proteins to be adsorbed on the surfaces thereof. As used herein, the term proteins includes any immunogenic substance, be it antigen or antibody. The bead material should be capable of establishing a hydrophobic bond with the protein so that the protein can be strongly adsorbed on the surface of the bead material. It has been found that plastic materials satisfy this criteria of adsorbing proteins from a properly prepared fluid medium which contacts the beads. The preferred bead material for purposes of the present invention is styrene acrylonitrile, although other plastic materials may be employed, such as nylon, polystyrene and the like.

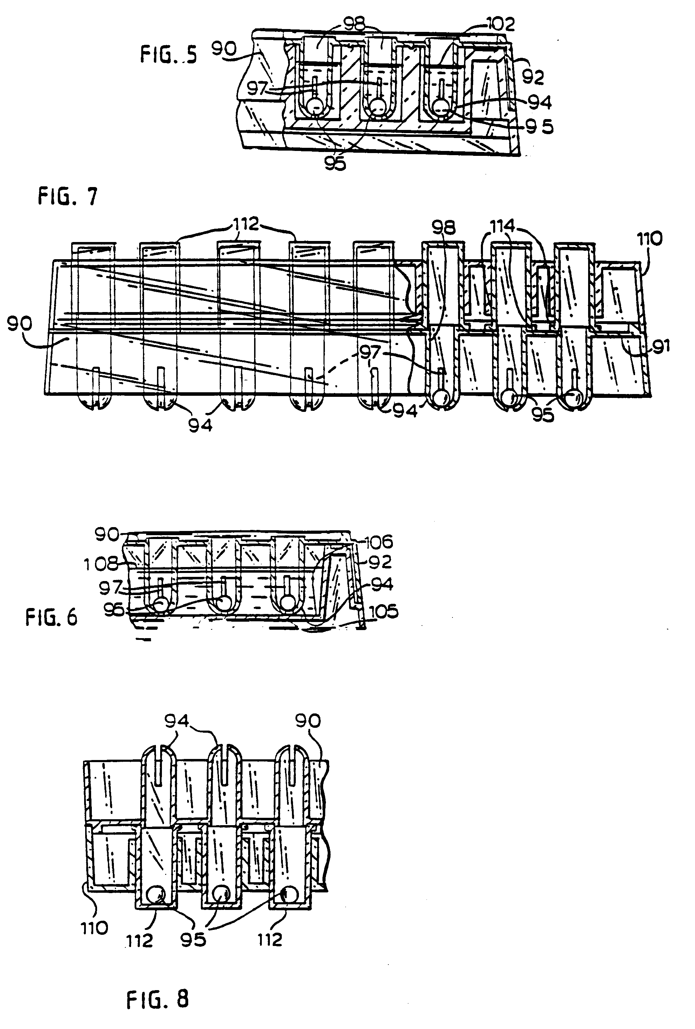

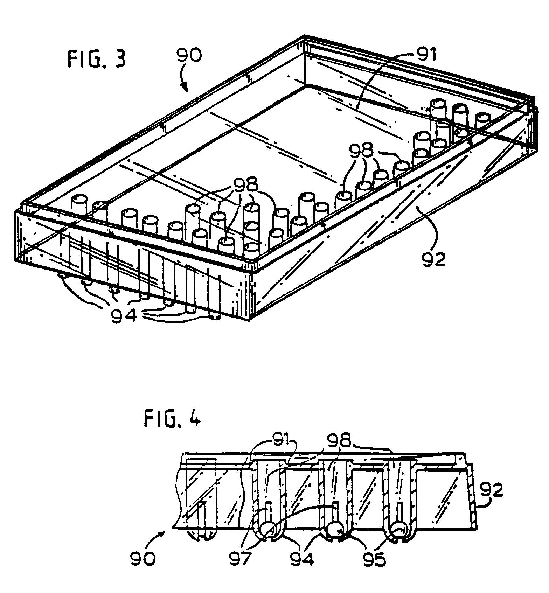

An embodiment of the present invention is illustrated in Figs. 3- 8. As can be seen by referring particularly to Figs. 3 and 4, this aspect of the invention comprises a carrier plate 90. This plate includes a generally planar support body 91 with a downwardly depending wall 92 surrounding body 91. Also depending downwardly from body 91 is a plurality of separated, individualized pockets 94. These pockets are large enough to hold a bead 95 such as described above in relation to Figs 1 and 2, above. In this embodiment, the pockets are orthogonally arranged in rows and columns, with there being preferably 96 such pockets. Each pocket includes one or more slots 97 in its distal end which is the end of the pocket to be placed into a well of a tray as hereinafter described. Slot 97 has a transverse dimension smaller than the diameter of bead 95 so that fluid can enter into the pocket, but the bead will be maintained in the bottom of the pocket during use. At the opposite end of each pocket is a larger opening 98, which is large enough to deposit and subsequently remove the bead from the pocket.

Carrier plate 90 is seen positioned on a tray or plate 100 which is substantially similar to the trays previously described, having separated, individualized wells 101. Each pocket depends into one of the wells containing fluid 102 therein. This fluid enters into the pocket by passing through the slot 97 in each pocket and bathes bead 95 contained in the pocket. Beads 95 are adapted to adsorb proteins on their surfaces and are preferably made of the materials heretofore described. It can be seen in Fig. 5 that the level of fluid 102 can be maintained so that no fluid will exit from the pocket through larger opening 98 therein.

The carrier plate embodiment of Figs. 3 and 4, of course, can be utilized with many different types of fluid carrying plates or trays. One such different tray is illustrated in Fig. 6, wherein tray 105 has one continuous cup-shaped recess 106. Fluid 108 placed in recess 106 will then bathe all of the beads 95 simultaneously. This type of tray 105 may be used for various washings of the beads during the various steps of the immunoassay procedures.

After beads 95 have been treated during the immunoassay procedures, it is desirable to remove them from the carrier plate and place them in individual test tubes for further testing, such as the RIA procedure. To accomplish this, carrier plate 90 is removed from the tray containing the fluid as heretofore described. A rack 110 containing a number of test tubes 112 corresponding in number to pockets 94 is placed over body 91 of carrier plate 90. The open ends 114 of test tubes 112 are aligned over larger openings 98 in the pockets, so that communication is established between the pockets and the test tubes, as more clearly seen in Fig. 7. Once rack 110 is placed onto carrier plate 90 with the test tubes aligned with pockets 94, a quick inversion of the composite assembly will cause beads 95 to drop from the pockets into the test tubes, as illustrated in Fig. 8. Test tubes 112 may then be removed individually to screen in RIA equipment.

While the invention as described herein can be used by researchers for the detection and quantification through the immunoassay method, of many different biological substances, it finds significant application in the testing for monoclonal antibodies and, therefore, for specific hybridoma cells. Specifically, a simple test may be performed using the present invention to make a determination in which of the wells there are antibody-producing hybridoma cells. Such a determination is possible due to the interaction between antibody and antigen and the manner of protein treatment of the beads, disks or other bodies which adsorb immunogenic substances thereto. A more complete screening test utilizing the present invention can determine which of the wells that contain hybridoma cells also contain specific monoclonal antibody-producing hybridoma cells.

An example of the aforementioned simple test, to which the present invention, of course, is not limited, is as follows: a carrier plate in accordance with any one of the foregoing embodiments which includes beads, disks or other bodies thereon capable of adsorbing proteins, is positioned on a tray preferably containing 96 wells each containing hybridoma cells covered by HAT (containing Hypoxanthine, Aminopterin, Thymidin) fluid medium. If beads are utilized on the carrier plate, the medium then bathes the beads. This step may last between 15 and 45 minutes and is usually carried out at room temperature and under aseptic conditions. Following this bathing step, the carrier plate, with beads therein or thereon, is removed from the wells containing hybridoma cells, and positioned onto a tray, such as tray 105 illustrated in Fig. 6. This tray may be filled with a selected buffer solution into which the beads are rinsed by immersion. This immersion step may be repeated two or three times. Subsequent to the rinse, the carrier plate with treated beads will be immersed in another tray preferably containing 96 wells, each containing a solution of a chemical agent that will occupy all the sites on the beads that have not adsorbed antibodies from the hybridoma medium as described above. Examples of the chemical agents which may be utilized are ethanol-di-amine or polyvinylpyrrolydone. After this step, which is not mandatory and depends upon the circumstances which may last between 15 and 45 minutes, the beads are again rinsed in the same fashion as previously described. Thereafter, the carrier plate with the treated beads is positioned onto another tray preferably containing 96 wells. If, for example, the immunoassay procedure is an RIA test, each well could contain a medium known as 125I labeled-protein A, or preferably 125-I-labeled antimouse antibodies. After a rinse, the beads may be separated from the carrier plate and transported, by test tubes or the like, to a gamma counter in accordance with known RIA procedures. If, on the other hand, the ELISA procedure is to be utilized, the wells of the tray may be filled with an enzyme-labeled-protein A, or preferably enzyme-labeled anti-mouse antibodies. Following incubation and a rinsing procedure, the reacted beads (with or without separation from the carrier plate) can be placed in a tray which wells are filled with the enzyme substrate linked to a chromogen. After the color reaction develops, this latter tray's wells can be read in a spectrophotometer, or even visually, in accordance with known ELISA procedures. It is understood that other assay procedures may also utilize the multi-well assembly and components of the present invention.

Thus, the present invention provides a multi-well screening device which is useful in immunoassay procedures and which researchers may use in the simultaneous processing of a number of targets in the detection and quantification of biological substances.

**Claims**

1. A carrier plate (90) useful in immunoassay procedures and comprising:
   a generally planar support body (91);
   a plurality of separate, individualised pockets (94) in the body (91); and
   for each pocket, a removable body deposited in the respective pocket (94), each removable body being substantially spherical or disc shaped and having a surface adapted to bind an immunogenic substance;
   each pocket (94) including an upper opening (98) to allow deposit of the removable body into the respective pocket and to allow removal of the removable body from the pocket, and each pocket (94) further including a lower opening (97) which is smaller than the removable body deposited in the pocket (94) and which allows fluid to enter into the pocket (94) while the removable body (95) is in the pocket.

2. A carrier plate according to claim 1, wherein the lower opening (97) in each pocket (94) takes the form of one or more slots.

3. A carrier plate according to claim 1 or 2, wherein the pockets are arranged in orthogonal columns and rows.

4. A carrier plate according to claim 3 which has 96 pockets.

5. A carrier plate according to any preceding claim, wherein the pockets (94) depend downwardly from the support body (91).

6. A carrier plate according to any one of claims 1 to 5 wherein the removable body (95) in each pocket is a substantially spherical plastics bead.

7. A screening assembly useful in immunoassay procedures and comprising:

a carrier plate (90) as defined in any preceding claim; and

a tray (100) adapted to hold fluid and adapted to receive the carrier plate in a disposition such that the removable beads can be contacted with fluid placed in the tray.

8. An assembly according to claim 7 wherein the tray (100) has a plurality of separate, individualised wells (101) arranged to align with the pockets so that there is one well for each pocket.

9. Use of an assembly according to claim 7 or 8 as part of the equipment for a radioimmunoassay (RIA) for screening of hybridoma cultures for antibody production, the removable bodies (95) having been contacted with the hybridoma cultures.

## Revendications

1. Une plaque de support (90) utile dans les modes opératoires des analyses immunologiques comprenant:

un corps de support (91) généralement plan;

une pluralité de poches séparées et individualisées (94) dans le corps (91) et pour chaque poche, un corps détachable déposé dans la poche respective (94), chaque corps détachable étant sensiblement sphérique ou en forme de disque et présentant une surface adaptée à la fixation d'une substance immunogène;

chaque poche (94) comprenant une ouverture supérieure (98) pour permettre le dépôt du corps détachable dans la poche respective et pour permettre l'enlèvement du corps détachable de la poche et chaque poche (94) comprenant en outre une ouverture inférieure (97) qui est plus petite que le corps détachable déposé dans la poche (94) et qui permet au fluide de pénétrer dans la poche (94) alors que le corps détachable (95) se trouve dans la poche.

2. Une plaque de support suivant la revendication 1, dans laquelle l'ouverture inférieure (97) dans chaque poche prend la forme d'une ou de plusieurs rainures.

3. Une plaque de support suivant la revendication 1 ou 2, dans laquelle les poches sont disposées en colonnes et en rangées orthogonales.

4. Une plaque de support suivant la revendication 3 qui a 96 poches.

5. Une plaque de support suivant l'une quelconque des revendications précédentes, dans laquelle les poches (94) pendent vers le bas à partir du corps du support (91).

6. Une plaque de support suivant l'une quelconque des revendications 1 à 5, dans laquelle le corps détachable (95) dans chaque poche est une perle sensiblement sphérique en matière plastique.

7. Un assemblage pour criblage utile dans les modes opératoires des analyses immunologiques et comprenant:

une plaque de support (90) telle que définie dans l'une quelconque des revendications précédentes et

un plateau (100) adapté de manière à contenir un fluide et adapté de manière à recevoir la plaque de support dans une disposition telle que les perles détachables puissent être mises en contact avec le fluide déposé dans le plateau.

8. Un assemblage suivant la revendication 7, dans lequel le plateau (100) a une pluralité de puits séparés et individualisés (101) disposés de manière à s'aligner avec les poches de sorte qu'il y ait un puits pour chaque poche.

9. Utilisation d'un assemblage suivant la revendication 7 ou 8 comme partie de l'équipement pour un radioimmunoessai (RIA) pour le criblage de cultures d'hybridomes pour la production d'anticorps, les corps détachables (95) ayant été mis en contact avec les cultures d'hybridomes.

## Patentansprüche

1. Trägerplatte (90), die für immunologische Tests nützlich ist, mit:

einem im allgemeinen ebenen Stützkörper (91);

einer Mehrzahl von getrennten, einzelnen Taschen (94) in dem Körper (91); und

mit einem für jede Tasche in der jeweiligen Tasche (94) angeordneten, herausnehmbaren Körper, wobei jeder herausnehmbare Körper im wesentlichen kugelförmig oder scheibenförmig ist und eine Oberfläche besitzt, die geeignet ist, eine Immunsubstanz zu binden;

wobei jede Tasche (94) einen obere Öffnung (98) umfaßt, um die Ablage des herausnehmbaren Körpers in die jeweilige Tasche und das Herausnehmen des herausnehmbaren Körpers aus der Tasche zu erlauben, und wobei jede Tasche (94) außerdem eine untere Öffnung (97) aufweist, die kleiner als der in der Tasche (94) angeordnete, herausnehmbare Körper ist und die das Eindringen eines Fluids in die Tasche (94) erlaubt, während sich der herausnehmbare Körper (95) in der Tasche befindet.

2. Trägerplatte nach Anspruch 1, bei der die untere Öffnung (97) in jeder Tasche (94) die Form von einem oder mehreren Schlitzen besitzt.

3. Trägerplatte nach Anspruch 1 oder 2, bei der die Taschen in rechtwinkligen Zeilen und Spalten angeordnet sind.

4. Trägerplatte nach Anspruch 3, die 96 Taschen besitzt.

5. Trägerplatte nach einem der vorhergehenden Ansprüche, bei der die Taschen (94) von dem Stützkörper (91) nach unten hängen.

6. Trägerplatte nach einem der Ansprüche 1 bis 5, bei der der herausnehmbare Körper (95) in jeder Tasche eine im wesentlichen kugelförmige Plastikperle ist.

7. Klassifizierungsanordnung, die für immunologische Tests nützlich ist und umfaßt:

eine Trägerplatte (90) nach einem der vorhergehenden Ansprüche; und

ein Tablett (100), das geeignet ist, ein Fluid zu halten, und das geeignet ist, die Trägerplatte in einer solchen Anordnung zu halten, daß die herausnehmbaren Perlen mit dem auf dem Tablett befindlichen Fluid in Kontakt gebracht werden können.

8. Anordnung nach Anspruch 7, bei der das Tablett (100) eine Mehrzahl von getrennten, unabhängigen Vertiefungen (101) besitzt, die so angeordnet sind, daß sie mit den Taschen ausgerichtet sind, so daß es eine Vertiefung für jede Tasche gibt.

9. Verwendung einer Anordnung nach Anspruch 7 oder 8 als Teil einer Ausrüstung für eine Radioimmununtersuchung (RIA) zum Klassifizieren von Hybridomakulturen zur Antikörpererzeugung, wobei die herausnehmbaren Körper (95) mit den Hybridomakulturen kontaktiert wurden.

FIG.1

FIG.2

FIG.2a

FIG. 5

FIG. 7

FIG. 6

FIG. 8

FIG. 3

FIG. 4